# EUROPEAN PATENT SPECIFICATION

(11) **EP 2 568 972 B1**
(45) Date of publication and mention of the grant of the patent: **02.08.2017**
(21) Application number: 11780838.6
(22) Date of filing: 13.05.2011
(51) Int. Cl.: A61K 9/24, A61K 9/20, A61K 31/415, A61K 31/40, A61P 3/06, A61P 3/00, A61K 9/16, A61K 45/06

(54) **PHARMACEUTICAL FORMULATION IN THE FORM OF BILAYERED TABLETS COMPRISING HMG-COA REDUCTASE INHIBITOR AND IRBESARTAN**
PHARMAZEUTISCHE FORMULIERUNG IN FORM ZWEISCHICHTIGER TABLETTEN MIT EINEM HMG-COA-REDUKTASEHEMMER UND IRBESARTAN
FORMULATION PHARMACEUTIQUE SOUS LA FORME DE COMPRIMÉS BICOUCHES COMPRENANT UN INHIBITEUR DE HMG-COA RÉDUCTASE ET D'IRBÉSARTAN

(30) Priority: 08.06.2010 KR 20100053782; 14.05.2010 KR 20100045636
(43) Date of publication of application: 20.03.2013
(73) Proprietor: Hanmi Science Co., Ltd., Gyeonggi-do 445-813 (KR)
(72) Inventor: KIM, Yong Il, Suwon-si Gyeonggi-do 442-718 (KR); NA, Young Jun, Suwon-si Gyeonggi-do 442-150 (KR); KIM, Min Jung, Gunpo-si Gyeonggi-do 435-040 (KR); KIM, Young-Hun, Suwon-si Gyeonggi-do 441-704 (KR); PARK, Jae Hyun, Suwon-si Gyeonggi-do 443-470 (KR); WOO, Jong Soo, Suwon-si Gyeonggi-do 440-710 (KR)
(74) Representative: Goddar, Heinz J.
(86) International application number: PCT/KR2011/003549
(87) International publication number: WO 2011/142621

(56) References cited:
- WO-A2-2008/010008
- WO-A2-2008/068217
- KR-A- 20070 064 366
- KR-A- 20090 114 190
- KR-A- 20090 114 314
- KR-A- 20090 114 328
- KR-B1- 100 897 890
- US-A1- 2006 078 615
- US-A1- 2010 074 951

## Description

### FIELD OF THE INVENTION

The present invention relates to a pharmaceutical formulation in the form of a bilayered tablet comprising an HMG-CoA reductase inhibitor and irbesartan as active ingredients, which has an improved stability and dissolution rate.

### BACKGROUND OF THE INVENTION

Hyperlipidemia is the condition of abnormally elevated levels of lipids such as cholesterols, triglycerides, and others, in the plasma. Hyperlipidemia, particularly hypercholesterolemia, induces arterial thrombosis, resulting in arteriosclerosis in which an artery wall thickens as the result of accumulation of lipids. Arteriosclerosis is clinically important since it can lead to cardiovascular diseases such as ischemic heart disease, angina pectoris, and myocardial infarction. The prevention of arteriosclerosis may be achievable by way of the treatment of hypercholesterolemia highly associated therewith.

Hyperlipidemia or elevated level of lipids in plasma is associated with the increased incidence frequency of cardiovascular diseases and arteriosclerosis. More specific types of hyperlipidemia may include hypercholesterolemia, familial dysbetalipoproteinemia, diabetic dyslipidemia, dyslipidemia linked to nephropathy, familial combined hyperlipidemia, and others. Hypercholesterolemia results in elevated levels of LDL-cholesterol and total cholesterol in plasma. LDL transports cholesterol in blood. In addition, familial dysbetalipoproteinemia, also known as type III hyperlipidemia, is characterized by the accumulation of beta VLDL (very low density lipoprotein) in plasma. Further, this symptom is involved in the substitution of a normal apolipoprotein E3 with an abnormal isoform, apolipoprotein E2. Diabetic dyslipidemia results in a multiple of lipoprotein disorders including overproduction of VLDL-cholesterol, abnormal lipolysis of VLDL triglycerides, decreased activity of LDL-cholesterol receptor, frequently occurring type III hyperlipidemia, and others. Dyslipidemia linked to nephropathy is hard to be treated and frequently occurring examples are hypercholesterolemia and hypertriglyceridemia. Familial combined hyperlipidemia is classified into multiple phenotypes of hyperlipidemia, i.e., type IIa, IIb, IV, V or hyperapobetalipoproteinemia.

For decades, HMG-CoA reductase inhibitors have been used to treat hyperlipidemia. These compounds have been known to lower total cholesterol and LDL-cholesterol in human body and to elevate HDL-cholesterol in some individuals. The conversion of HMG-CoA to mevalonate is an early and rate-limiting step in the biosynthesis of cholesterol. The inhibition of HMG-CoA reductase which blocks the production of mevalonate is accomplished based on that HMG-CoA reductase inhibitors show the reduction effects on total cholesterols and on LDL-cholesterols (Grundy S. M., N. Engl. J. Med., 319(1):24-32, 25-26, 31(1988)).

Examples of HMG-CoA reductase inhibitors include mevastatin (U.S. Pat. No. 3,983,140), lovastatin (also called mevinolin; U.S. Pat. No. 4,231,938), pravastatin (U.S. Pat. Nos. 4,346,227 and 4,410,629), pravastatin lactone (U.S. Pat. No. 4,448,979), velostatin (also called synvinolin; U.S. Pat. Nos. 4,448,784 and 4,450,171), simvastatin, rivastatin, fluvastatin, atorvastatin, rosuvastatin, cerivastatin, and others.

According to the U.S. Food and Drug Administration (FDA) Summary Basis of Approval (SBA) for Warner-Lambert's Lipitor^{™}, atorvastatin is present in multiple amorphous and crystalline forms. Originally, atorvastatin is synthesized in the amorphous form, but it has been reported that this form is hygroscopic and unstable when exposed to oxygen. On the other hand, a crystalline form of atorvastatin developed later shows an improved *in vivo* absorption rate (i.e., an approximate 50% increase in Cmax), but is nevertheless highly susceptible to heat, moisture, a low pH environment, and light, which requires attention in selecting excipients or additives in the product development.

Irbesartan, chemically known as 3-butyl-3-((4-(2-(2-tetrazol-5-yl)phenyl)phenyl)methyl)-1,3-diazaspiro(4,4)non-1-en-4-one, is an angiotensin-II-receptor antagonist, which blocks angiotensin II, one of substances causing vascular constriction, from binding with AT1 and thus exhibits an antihypertensive effect. It selectively blocks AT1 receptors, but allows angiotensin II to bind with AT2 receptor, thereby inhibiting endothelial proliferation, vasoconstriction, and tissue repair while maintaining vasodilatation.

These commercial available angiotensin-II-receptor antagonists have been extensively used as hypertension treatment drugs for the past several years. Their effects have been demonstrated through clinical trials [Pharmacologic, pharmacokinetic, and therapeutic difference among angiotensin-II-receptor antagonist: Pharmacotherapy 20(2):130-139, 2000]. In US2010/0074951 a bilayered tablet comprising Irbesartan in one layer and a HMG-CoA reductase inhibitor in another layer is disclosed. These angiotensin-II-receptor antagonists have been known to be efficacious in preventing or treating heart failure associated with various symptoms of hypertension, post-myocardial infarction arrhythmia and heart failure, diabetic complications, renal failure, and stroke. Further, they are known to have another effects, such as an antiplatelet effect, prevention of arteriosclerosis, inhibition of the adverse effects of aldosterone, relief metabolic syndrome symptoms, and prevention of circulatory diseases aggravation [J.Wagner et al., Effects of AT1 receptor blockade on blood pressure and the renin angiotensin system in spontaneously hypertensive rats of the stroke prone strain, Clin, Exp. Hypertens., vol.20(1998), p.205-221; M. Bohm et al., Angiotensin-II-receptor blockade in TGR(mREN2)27 : Effects of renin-angiotensin-system gene expression and cardiovascular functions, J. Hypertens., vol.13(8)(1995), p.891-899].

Irbesartan is a fluffy material having relatively low bulk and tap densities. Further irbesartan is stick and can adhere to surfaces such as tablet punch faces and dies, causing problems in tableting. In addition, as irbesartan has a low aqueous solubility, i.e., solubility in water, it is essential to use a surfactant to enhance the wetting or solubility of a tablet (Korean Patent No. 0442719).

When the angiotensin-II-receptor antagonist is used in combination with an HMG-CoA reductase inhibitor, not only is it more effective to treat hypertension and hyperlipidemia, compared to each single agent, but diabetes can also be treated by the results of strengthened blood vessel endothelial cells (a protective membrane) and increased insulin sensitivity.

In addition, it has been demonstrated that about 60% of patients with hypertension also suffer from hyperlipidemia, and hypertension and hyperlipidemia are closely correlated with each other. The co-administration of both drugs to patients with cardiovascular diseases is highly effective in reducing the occurrence of complications such as stroke and death from stroke, and in preventing diabetes [Circulation, May 2005; 111: 2518-2524, Circulation, Dec 2004; 110: 3687 - 3692].

Complex formulations of irbesartan and atorvastatin are disclosed in Korean Patent Publication Nos. 2009-0114328 and 2009-0114190. The complex formulations allow a delayed-release of one of two drugs over 2 hours, for the purpose of preventing the interaction between ARBs including irbesartan and an HMG-CoA reductase inhibitor. However, the delayed release formulations were designed only for in vitro test, such as a dissolution tester, and it is difficult to prepare a product having a constant delayed release rate by using the same. In addition, due to the difference in individual gastrointestinal movements, it is also hard to anticipate the delayed release time precisely. Furthermore, irbesartan is known to be mostly metabolized by 2C9 of cytochrome P450, a hepatic metabolic enzyme, while HMG-CoA reductase inhibitors such as losuvastatin, pitavastatin and pravastatin are little metabolized by the liver, and HMG-CoA reductase inhibitors such as atorvastatin, lovastatin and simvastatin are mostly metabolized by 3A4 of cytochorme P450, which indicates little or no possibility of correlation between irbesartan and HMG-CoA reductase inhibitors [Pharmacology & Therapeutics, Vol. 112, Issue 1, October 2006; 71-105, FDA Avapro label].

Therefore, when two drugs in a complex formulation has no correlation with each other, it is deemed that an immediate release formulation, which shows efficacies of the two drugs within a short period of time, is desirable, and the present inventors have thus completed the invention by developing an immediate release formulation containing an HMG-CoA reductase inhibitor and irbesartan as active ingredients, which has an improved stability and dissolution rate.

### SUMMARY OF THE INVENTION

Therefore, it is an object of the present invention to provide a complex formulation of an HMG-CoA reductase inhibitor and irbesartan, which has an improved stability due to minimized, physical and chemical interactions between irbesartan and an HMG-CoA reductase inhibitor, and exhibits immediate release properties for the two drugs, and an improved solubility and bioavailability of irbesartan.

In accordance with the present invention, there is provided a pharmaceutical formulation in the form of a bilayered tablet comprising: a) a first layer containing irbesartan or a pharmaceutically acceptable salt thereof; and b) a second layer containing an HMG-CoA reductase inhibitor and a basic additive, wherein the basic additive is contained in the second layer only; and wherein the basic additive is NaHC03 , MgCO3 or a mixture thereof. The complex formulation of the present invention can improve the dissolution rate and stability of irbesartan and an HMG-CoA reductase inhibitor to enhance the bioavailability of the drug compared to conventional complex formulations and to minimize the generation of the related compounds, thereby being effectively used as a stable and superior therapeutic agent for hypertension and hypercholesterolemia.

### BRIEF DESCRIPTION OF THE DRAWINGS

The above and other objects and features of the present invention will become apparent from the following description of the invention, when taken in conjunction with the accompanying drawings, in which:
Fig. 1 is a graph showing the change in the amount of atorvastatin lactone, related compounds, after storage under accelerated conditions (40°C, 75% RH) for the formulations prepared in Examples and Comparative Examples;
Fig. 2 is a graph showing the change in the amount of degradation products of irbesartan (RRT 0.8) after storage under accelerated conditions (40°C, 75% RH) for the formulations prepared in Examples and Comparative Examples;
Fig. 3 is a graph showing the change in the amount of related compounds after storage under accelerated conditions (40°C, 75% RH) for the single tablets prepared in Comparative Examples;
Fig. 4 is a graph showing the dissolution rate of irbesartan for the formulations prepared in Examples and Comparative Examples, and for a commercially available formulation (Aprovel);
Fig. 5 is a graph showing the dissolution rate of atorvastatin for the formulations prepared in Examples and Comparative Examples, and for a commercially available formulation (Lipitor);
Fig. 6 is a graph showing the saturation solubility of irbesartan for the formulations prepared in Examples and Comparative Examples;
Fig. 7 is a graph showing the change in the bioavailability of irbesartan for the formulations prepared in Examples and Comparative Examples; and
Fig. 8 is a schematic diagram of an exemplary pharmaceutical formulation in the forms of a bilayered tablet according to the present invention.

### DETAILED DESCRIPTION OF THE INVENTION

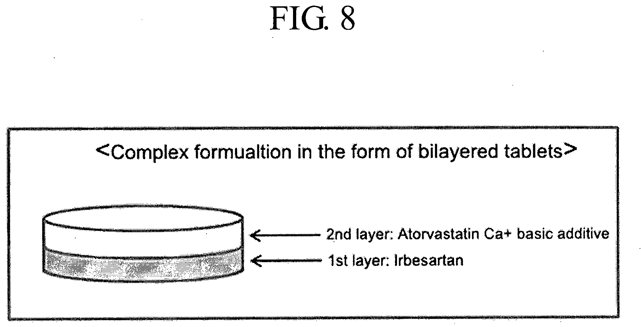

The complex formulation according to the present invention is characterized by a bilayered tablet consisting of a first layer containing irbesartan or a pharmaceutically acceptable salt thereof and a second layer containing an HMG-CoA reductase inhibitor and a basic additive. An example of the pharmaceutical formulation in the form of a bilayered tablet is depicted in Fig. 8. Hereinafter, the properties and types of the components included in the complex formulation of the present invention are described in detail.

### (i) First layer

In the complex formulation in the form of a bilayered tablet according to the present invention, the first layer may contain irbesartan or a pharmaceutically acceptable salt thereof.

Irbesartan, i.e., 2-n-butyl-4-spirocyclopentan-1-[(2'-(tetrazol-5-yl)biphenyl-4-tl)methyl]-2-imidazolin-5-one, is a potential long-term acting angiotensin-II-receptor antagonist, which binds to angiotensin receptors with a high affinity to inhibit the vasoconstriction, the aldosterone excretion and the resorption of moisture and sodium, and thus exhibits an antihypertensive effect. Therefore, it is particularly useful in treating cardiovascular diseases such as hypertension and heart failure. Irbesartan is represented by Formula (I), as described in U.S. Patent No. 5,270,317.

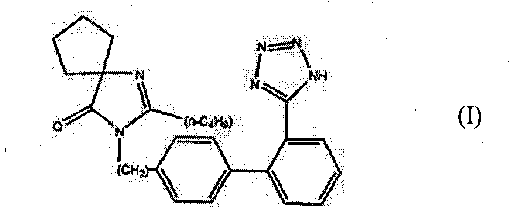

Pharmaceutically acceptable salts of irbesartan are well known in the art.

The complex formulation according to the present invention may preferably comprise irbesartan or a pharmaceutically acceptable salt thereof in an amount of 8 mg to 600 mg per unit dosage form.

The first layer may further comprise a surfactant for ameliorating the hydrophobicity of irbesartan. The surfactant improves the aqueous granulation of irbesartan, eases the release of tablets after compression, and accelerates the dissolution of irbesartan active ingredients. Representative examples of surfactants being used include, but not limited to, sodium lauryl sulfate, poloxamer, polyethylene glycol, and mixtures thereof, particularly poloxamers. In an embodiment of the present invention, the surfactant is preferably present only in the first layer for improving stability, but not limited thereto.

In addition, the first layer may further comprise binders, disintegrants, lubricants, or mixtures thereof, and any other excipeints and adjuvants. The binders may be at least one selected from the group consisting of alginic acid, sodium alginate, sodium carboxymethylcellulose, ethylcellulose, hydroxyethylcellulose, hydroxypropylcellulose, hydroxypropylmethylcellulose, methylcellulose, gelatin, povidone, starch, pregelatinized starch and mixtures thereof. The disintegrants may be at least one selected from the group consisting of alginic acid, sodium alginate, sodium carboxymethylcellulose, microcrystalline cellulose, powdered cellulose, croscarmellose sodium, crospovidone, pregelatinized starch, sodium carboxyl methyl starch, starch and mixtures thereof. The lubricants may be at least one selected from the group consisting of calcium stearate, glyceryl monostearate, glyceryl palmitostearate, magnesium stearate, sodium lauryl sulfate, sodium stearyl fumarate, zinc stearate or stearic acid, hydrogenated vegetable oil, polyethylene glycol, sodium benzoate, talc, and mixtures thereof, but not limited thereto.

Further, the other excipients and adjuvants may be diluents, coloring agents, antiadherents, or mixtures thereof, but not limited thereto.

In a preferred embodiment, the first layer containing irbesartan may comprise (a) irbesartan in an amount of about 20% to about 70% by weight, more preferably 40% to 60% by weight, (b) diluents in an amount of about 1% to about 70% by weight, (c) binders in an amount of about 2% to about 20% by weight, (d) disintegrants in an amount of about 1 % to about 10% by weight, (e) antiadherents in an amount of about 0.1% to about 5% by weight, (f) lubricants in an amount of about 0.2% to 5% by weight, and (g) coloring agents in an amount of 2% or less by weight, more preferably about 0.1 % to 1 % by weight, based on the weight of the first layer.

### (ii) Second layer

The second layer of the bilayered complex formulation according to the present invention contains an HMG-CoA reductase inhibitor and a basic additive, wherein the basic additive is contained in the second layer only; and wherein the basic additive is NaHC03, MgCO3 or a mixture thereof. The HMG-CoA reductase inhibitor is a drug capable of preventing or treating hyperlipidemia and arteriosclerosis by reducing the level of lipoproteins or lipids in blood, and particular examples thereof are rosuvastatin, lovastatin, atorvastatin, pravastatin, fluvastatin, pitavastatin, simvastatin, rivastatin, cerivastatin, velostatin, mevastatin, and pharmaceutically acceptable salts, precursors or mixtures thereof, more preferably atorvastatin, but not limited thereto.

The complex formulation according to the present invention contains an HMG-CoA reductase inhibitor, preferably in an amount of 0.5 mg to 100 mg, more preferably 2.5 mg to 80 mg, most preferably 5mg to 80mg, per unit dosage form, but not limited thereto. The inventive complex formulation includes NaHCO₃, MgCO₃ and mixtures thereof, but not limited thereto. The basic additives should be present in the same layer with the HMG-CoA reductase inhibitor, to improve the stability of the HMG-CoA reductase inhibitor and provide basic microenvironment conditions that enhance the solubility of irbesartan, ultimately increasing the bioavailability of irbesartan.

The basic additive may be used in an amount of 2 to 10 parts by weight based on 1 part of HMG-CoA reductase inhibitor, and in an amount of 0.2 to 10 parts by weight based on 1 part of irbesartan.

In addition, the second layer may further comprise water-soluble diluents and optionally other excipients and adjuvants. The water-soluble diluents may be at least one selected from the group consisting of mannitol, sucrose, lactose, sorbitol, xylitol, glucose, and mixtures thereof, but not limited thereto. Further, the excipients and adjuvants may be disintegrants, binders, carriers, fillers, lubricants, rheology modifiers, crystallization retarders, solubilizers, coloring agents, pH modifiers, surfactants, emulsifiers, coating agents, or mixtures thereof, but not limited thereto.

Examples of the disintegrants include hydroxypropylcellulose, crospovidone, sodium starch glycolate, croscarmellose sodium, and others, and may be appropriately, selected from conventionally available disintegrants. Examples of the binders are povidone, copovidone, cellulose, and others. In addition, examples of the lubricants are magnesium stearate, sodium stearyl fumarate, talc, glycerin fatty acid ester, glycerol dibehenate, and others, and may be appropriately selected from conventionally available lubricants. Further, examples of the coating agents are polyvinyl alcohol, hydroxypropylmethylcellulose, methylcellulose, ethylcellulose, and others, and may be appropriately selected from conventionally available coating agents.

In a preferred embodiment, the second layer may contain an HMG-CoA reductase inhibitor in an amount of about 5 to about 20% by weight, more preferably about 6 to about 9% by weight, components for preparation of granules such as diluents, disintegrants and binders in an amount of about 2 to about 70% by weight, more preferably 2 to 20 by weight, lubricants or coating agents in an amount of about 0.5 to 2% by weight, more preferably 0.7 to 1.5 by weight, and additives in an amount of about 10 to 92.5% by weight, more preferably 15 to 80 by weight, based on the weight of the second layer.

### (iii) Bilayered tablet

The complex formulation according to the present invention is a bilayered tablet which consists of a first layer containing irbesartan or a pharmaceutically acceptable salt and a second layer containing an HMG-CoA reductase inhibitor and a basic additive, thus minimizing the contact between the drugs to improve the stability of each drug as well as the dissolution rate and wherein the basic additive is contained in the second layer only; and wherein the basic additive is NaHC03 , MgCO3 or a mixture thereof.

In particular, the pharmaceutical formulation in the form of a tablet according the present invention contains a basic additive in a second layer, and the additive used can improve not only the stability of the HMG-CoA reductase inhibitor, but also the stability of irbesartan by minimizing the contact between irbesartan and basic additives.

Further, the additives may improve the dissolution rates of the two drugs, thereby ameliorating the drawbacks such as low stability and dissolution rate in complex tablets. For example, the pharmaceutical formulation in the form of a bilayered tablet according to the present invention may exhibit a dissolution profile such that 80% or more of each of irbesartan and an HMG-CoA reductase inhibitor is released within 30 minutes, preferably 80% or more within 15 minutes.

The pharmaceutical formulation in the form of a bilayered tablet containing HMG-CoA reductase inhibitor and irbesartan may be prepared by a process comprising the steps of:
(i) granulating irbesartan or a pharmaceutically acceptable salt thereof to obtain granules for a first layer;
(ii) granulating a mixture of an HMG-CoA reductase inhibitor and a basic additive (wherein the basic additive is NaHC03, MgCO3 or a mixture thereof) to obtain granules for a second layer; and
(iii) compressing the granules for the first layer and the second layer into a bilayered tablet.

The various processes involved in the preparation of the complex formulation according to the present invention may be performed based on conventional processes.

In an embodiment of the present invention, the granulation process may comprise following steps:
(a) blending irbesartan or atorvastatin with preferred disintegrants and optionally some or all of excipients necessary for a final composition;
(b) adding granulating solvents to the mixture obtained in step (a) under shear conditions;
(c) optionally, pulverizing, milling, or sieving the resultant obtained in step (b), followed by drying the wet material through air drying, fluid bed drying, oven drying
(d) optionally, pulverizing or sieving the material obtained in step (c);
(e) blending the composition thus obtained with one or more disintegrants, and optionally additional excipients preferably including lubricants; and
(f) molding the final composition into granules.

In step (a), the excipient may contain diluents, binders, and other substances necessary for improving fluidity and stability or processing and formation of unit dosage forms. In step (b), preferred granulating solvents include water, ethanol, isopropanol, and mixtures thereof. Other components (e.g., binders, wetting agents, buffers, etc.) known in the art may be added to the granulating solvent. Various methods known in the art, based on high shear granulation, low shear granulation, fluid bed granulation, compression granulation, and other may be used in step (b). In step (c), the drying may be carried out, preferably at a temperature not exceeding about 60°C, more preferably at a temperature not exceeding about 50°C, most preferably at a temperature not exceeding about 40°C.

The complex formulation of the present invention can improve the dissolution rate and stability of irbesartan and HMG-CoA reductase inhibitors to enhance the bioavailability of the drug compared to conventional complex formulations and to minimize the generation of related compounds, thereby being effectively used as a stable and superior therapeutic agent for hypertension and hypercholesterolemia.

The following Examples are provided to illustrate preferred embodiments of the invention.

### Preparation Example 1-1: Preparation of granules comprising irbesartan

In accordance with the composition described in Table 1, irbesartan (Hanmi Fine Chemical Co., Ltd., Korea), mannitol, pregelatinized starch, and croscarmellose sodium (DMV international) were mixed, and the mixture was then kneaded with a binding solution of povidone (BASF, Germany) dissolved in water, dried, and sieved through a 30 mesh to obtain wet granules, followed by addition of magnesium stearate and mixing to prepare irbesartan granules.

### Preparation Example 1-2: Preparation of granules comprising irbesartan

In accordance with the composition described in Table 1, irbesartan (Hanmi Fine Chemical Co., Ltd., Korea), mannitol, pregelatinized starch, and croscarmellose sodium (DMV international) were mixed, and the mixture was then kneaded with a binding solution of povidone (BASF, Germany) and poloxamer 188 (BASF, Germany) dissolved in water, dried, and sieved through a 30 mesh to obtain wet granules, followed by addition of magnesium stearate and mixing to prepare irbesartan granules.

**<Table 1>**

| Granules comprising irbesartan (unit: mg) | | |
|---|---|---|
| Ingradients | Prep. Ex. 1-1 | Prep. Ex. 1-2 |
| Irbesartan | 150 | 150 |
| Mannitol | 47 | 47 |
| Pregelatinized starch | 23 | 23 |
| Croscarmellose sodium | 12 | 12 |
| Povidone | 8 | 8 |
| Poloxamer 188 | | 9 |
| <Water> | <80> | <80> |
| Magnesium stearate | 4 | 4 |
| Total | 244 | 253 |

### Preparation Example 2-1: Preparation of granules comprising atorvastatin

In accordance with the composition described in Table 2, atorvastain calcium (TEVA, India), mannitol, microcrystalline cellulose, and crospovidone (BASF, Germany), and NaHCO₃ (Pendrice Soda, Australia) were mixed, and the mixture was then kneaded with a binding solution of HPC (hydroxypropylcellulose) and polysorbate 80 (Croda, USA) dissolved in water, dried, and sieved through a 30 mesh to obtain wet granules, followed by addition of magnesium stearate and mixing to prepare HMG-CoA reductase inhibitor granules.

### Preparation Example 2-2: Preparation of granules comprising atorvastatin

In accordance with the composition described in Table 2, atorvastatin calcium (TEVA, India), mannitol, microcrystalline cellulose, and crospovidone (BASF, Germany), and Magnesium carbonate (Tomita, Japan) were mixed, and the mixture was then kneaded with a binding solution of HPC and polysorbate 80 (Croda, USA) dissolved in water, dried, and sieved through a 30 mesh to obtain wet granules, followed by addition of magnesium stearate and mixing to prepare HMG-CoA reductase inhibitor granules.

### Preparation Example 2-3: Preparation of granules comprising atorvastatin

In accordance with the composition described in Table 2, atorvastatin calcium (TEVA, India), mannitol, microcrystalline cellulose, and crospovidone (BASF, Germany) were mixed, and the mixture was then kneaded with a binding solution of polysorbate 80 (Croda, USA) dissolved in water, dried, and sieved through a 30 mesh to obtain wet granules, followed by addition of magnesium stearate and mixing to prepare HMG-CoA reductase inhibitor granules.

**<Table 2>**

| Granules comprising atorvastatin (unit: mg) | | | |
|---|---|---|---|
| Ingredients | Prep. Ex. 2-1 | Prep. Ex. 2-2 | Prep. Ex. 2-3 |
| Atorvastatin calcium | 10.36 | 10.36 | 10.36 |
| Mannitol | 120 | 120 | 120 |
| Microcrystalline cellulose | 65.6 | 65.6 | 65.6 |
| Crospovidone | 36 | 36 | 36 |
| NaHCO₃ | 20 | | |
| Magnesium carbonate | | 100 | |
| HPC | 3 | 3 | 3 |
| Polysorbate 80 | 1.2 | 1.2 | 1.2 |
| <Water> | <300> | <300> | <300> |
| Magnesium stearate | 3 | 3 | 3 |
| Total | 259.26 | 339.16 | 239.16 |

### Examples 1 to 4: Preparation of a bilayered tablet of irbesartan-atorvastatin according to the present invention

Complex formulations in the form of a tablet comprising an HMG-CoA reductase inhibitor and irbesartan were prepared by combining granules of Preparation Examples as set forth in Table 3.

The irbesartan granules as a first layer and the HMG-CoA reductase inhibitor granules as a second layer were compressed into bilayered tablets using a tableting equipment to obtain complex formulations equivalent to irbesartan 150mg and HMG-CoA reductase inhibitor 10mg.

### Comparative Examples 1 to 13

### Comparative Examples 1 and 2: Preparation of bilayered tablets of irbesartan-atorvastatin containing no basic additive

The granules of Preparation Examples were combined and compressed into bilayered tablets containing irbesartan as a first layer and an HMG-CoA reductase inhibitor as a second layer, as set forth in Table 3.

### Comparative Examples 3 to 8: Preparation of monolayered tablets of irbesartan-atorvastatin

The granules of Preparation Examples were simply mixed and compressed into monolayered tablets, as set forth in Table 3.

### Comparative Examples 9 to 13: Preparation of single tablets

Each of granules of Preparation Examples 9 to 13 was compressed into a single tablet, as set forth in Table 3.

As described above, formulations of Comparative Examples 1 to 13 equivalent to irbesartan 150mg and/or HMG-CoA reductase inhibitor 10mg were prepared.

**<Table 3>**

| **Formulations comprising irbesartan and/or atorvastatin** | | | |
|---|---|---|---|
| | Type of tablet | Irbesartan | Atorvastatin |
| Ex. 1 | Bilayered | Prep. Ex. 1-1 | Prep. Ex. 2-1 |
| Ex. 2 | Bilayered | Prep. Ex. 1-1 | Prep. Ex. 2-2 |
| Ex. 3 | Bilayered | Prep. Ex. 1-2 | Prep. Ex. 2-1 |
| Ex. 4 | Bilayered | Prep. Ex. 1-2 | Prep. Ex. 2-2 |
| Comp. Ex. 1 | Bilayered | Prep. Ex. 1-1 | Prep. Ex. 2-3 |
| Comp. Ex. 2 | Bilayered | Prep. Ex. 1-2 | Prep. Ex. 2-3 |
| Comp. Ex. 3 | Monolayered | Prep. Ex. 1-1 | Prep. Ex. 2-1 |
| Comp. Ex. 4 | Monolayered | Prep. Ex. 1-1 | Prep. Ex. 2-2 |
| Comp. Ex. 5 | Monolayered | Prep. Ex. 1-1 | Prep. Ex. 2-3 |
| Comp. Ex. 6 | Monolayered | Prep. Ex. 1-2 | Prep. Ex. 2-1 |
| Comp. Ex. 7 | Monolayered | Prep. Ex. 1-2 | Prep. Ex. 2-2 |
| Comp. Ex. 8 | Monolayered | Prep. Ex. 1-2 | Prep. Ex. 2-3 |
| Comp. Ex. 9 | Single | Prep. Ex. 1-1 | |
| Comp. Ex. 10 | Single | Prep. Ex. 1-2 | |
| Comp. Ex. 11 | Single | | Prep. Ex. 2-1 |
| Comp. Ex. 12 | Single | | Prep. Ex. 2-2 |
| Comp. Ex. 13 | Single | | Prep. Ex. 2-3 |

### Experimental Example 1: Stability test

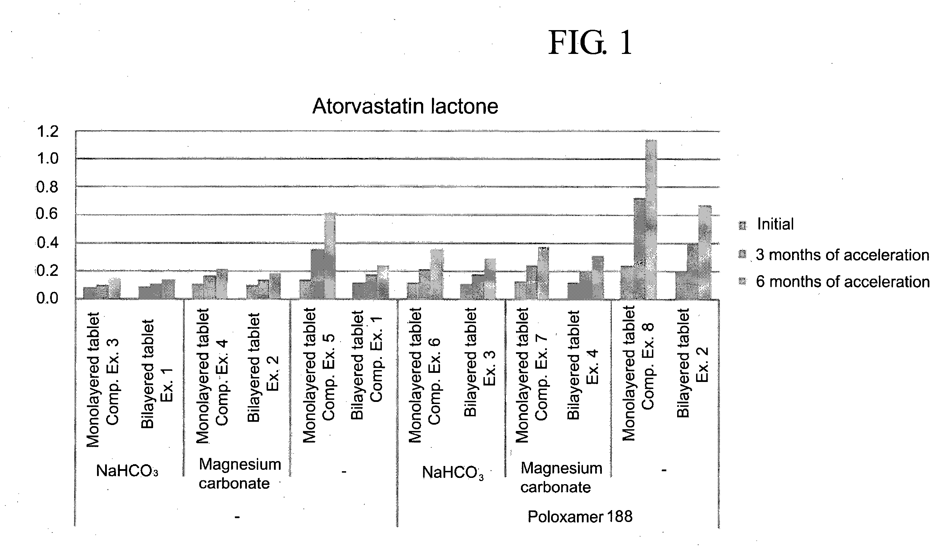

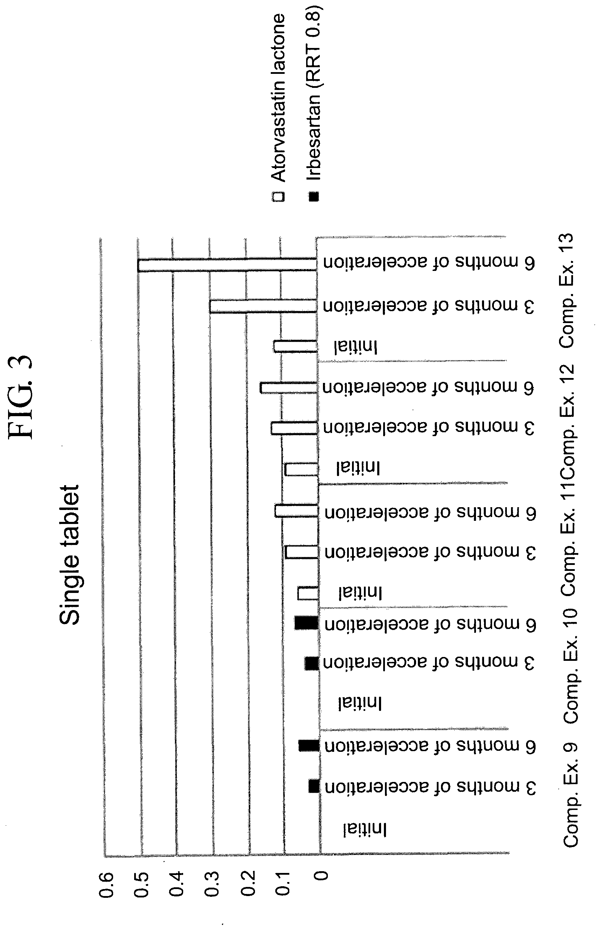

Complex formulations prepared in Examples 1 to 4 and Comparative Examples 1 to 8, and single formulations prepared in Comparative Examples 9 to 13 were each packaged with 1g of silica gel in an HDPE bottle, and stored under accelerated conditions (40°C, 75% RH) and measured for their stabilities three and six months later. The amount of degradation products of irbesartan (RRT 0.8) and the amount of atorvastatin lactone as a related compound, a representative acid degradation product, were measured. The results are shown in Tables 4 to 6 and Figs. 1 to 3.

**<Table 4>**

| Atorvastatin lactone after storage under accelerated conditions (40°C, 75% RH) | | | | | | |
|---|---|---|---|---|---|---|
| Surfactant | Basic additive | Example | | Initial | 3 months of acceleration | 6 months of acceleration |
| | NaHCO₃ | Monolayered | Comp. Ex. 3 | 0.08 | 0.10 | 0.15 |
| | | Bilayered | Ex. 1 | 0.09 | 0.11 | 0.14 |
| | Magnesium carbonate | Monolayered | Comp. Ex. 4 | 0.11 | 0.17 | 0.22 |
| | | Bilayered | Ex. 2 | 0.10 | 0.14 | 0.19 |
| | - | Monolayered | Comp. Ex. 5 | 0.14 | 0.36 | 0.62 |
| | | Bilayered | Ex. 1 | 0.12 | 0.18 | 0.25 |
| Poloxamer 188 | NaHCO₃ | Monolayered | Comp. Ex. 6 | 0.12 | 0.22 | 0.36 |
| | | Bilayered | Ex. 3 | 0.11 | 0.18 | 0.29 |
| | Magnesium carbonate | Monolayered | Comp. Ex. 7 | 0.13 | 0.25 | 0.38 |
| | | Bilayered | Ex. 4 | 0.12 | 0.21 | 0.31 |
| | - | Monolayered | Comp. Ex. 8 | 0.25 | 0.72 | 1.14 |
| | | Bilayered | Ex. 2 | 0.21 | 0.41 | 0.67 |

**<Table 5>**

| Degradation products of ibersartan (RRT 0.8) after storage under accelerated conditions (40°C, 75% RH) | | | | | | |
|---|---|---|---|---|---|---|
| Surfactant | Basic additive | Example | | Initial | 3 months of acceleration | 6 months of acceleration |
| | NaHCO₃ | Monolayered | Comp. Ex. 3 | 0.01 | 0.10 | 0.25 |
| | | Bilayered | Ex. 1 | 0.01 | 0.08 | 0.15 |
| | Magnesium carbonate | Monolayered | Comp. Ex. 4 | 0.02 | 0.11 | 0.23 |
| | | Bilayered | Ex. 2 | 0.01 | 0.07 | 0.16 |
| | - | Monolayered | Comp. Ex. 5 | 0.00 | 0.03 | 0.06 |
| | | Bilayered | Ex. 1 | 0.01 | 0.02 | 0.05 |
| Poloxamer 188 | NaHCO₃ | Monolayered | Comp. Ex. 6 | 0.02 | 0.11 | 0.24 |
| | | Bilayered | Ex. 3 | 0.01 | 0.07 | 0.16 |
| | Magnesium carbonate | Monolayered | Comp. Ex. 7 | 0.01 | 0.10 | 0.25 |
| | | Bilayered | Ex. 4 | 0.01 | 0.06 | 0.15 |
| | - | Monolayered | Comp. Ex. 8 | 0.01 | 0.04 | 0.07 |
| | | Bilayered | Ex. 2 | 0.00 | 0.02 | 0.05 |

**<Table 6>**

| Related compounds of single tablets after storage under accelerated conditions (40°C, 75% RH) | | | | | | |
|---|---|---|---|---|---|---|
| Example | | Basic additive | Surfactant | | Atorvastatin lactone | Degradation product of irbesartan (RRT 0.8) |
| Comp. Ex. 9 | Single tablet | | - | Initial | | 0.00 |
| | | | | 3 months of acceleration | | 0.03 |
| | | | | 6 months of acceleration | | 0.06 |
| Comp. Ex. 10 | | | Poloxamer 188 | Initial | | 0.00 |
| | | | | 3 months of acceleration | | 0.04 |
| | | | | 6 months of acceleration | | 0.07 |
| Comp. Ex. 11 | | NaHCO₃ | - | Initial | 0.06 | |
| | | | | 3 months of acceleration | 0.09 | |
| | | | | 6 months of acceleration | 0.12 | |
| Comp. Ex. 12 | | Magnesium carbonate | - | Initial | 0.09 | |
| | | | | 3 months of acceleration | 0.13 | |
| | | | | 6 months of acceleration | 0.16 | |
| Comp. Ex. 13 | | - | - | Initial | 0.12 | |
| | | | | 3 months of acceleration | 0.30 | |
| | | | | 6 months of acceleration | 0.50 | |

As shown in Tables 4 to 6 and Figs. 1 to 3, the amounts of atorvastain lactone and degradation products of irbesartan (RRT 0.8) had increased under accelerated conditions with times. In particular, the stability of a drug after 6 months under accelerated conditions is the critical factor in determining the shelf life of the drug. Related compounds should be not more than 0.2% for irbesartan and not more than 0.25% for atorvastatin, until 6 months of acceleration, based on the ICH guideline.

When the bilayered complex formulations of Example 1 to 4 and Comparative Examples 1 and 2 were compared as shown in Table 4 and Fig. 1, the experimental groups containing a basic additive such as NaHCO₃ or magnesium carbonate (Examples 1 and 2) showed more enhanced stability than the experimental group having no basic additive (Comparative Example 1), regarding the amount of atorvastain lactone generated. In addition, bilayered complex formulations (Examples 1 and 2) showed more enhanced stability than monolayered complex formulations (Comparative Examples 3 to 8), in terms of the amount of atorvastain lactone generated.

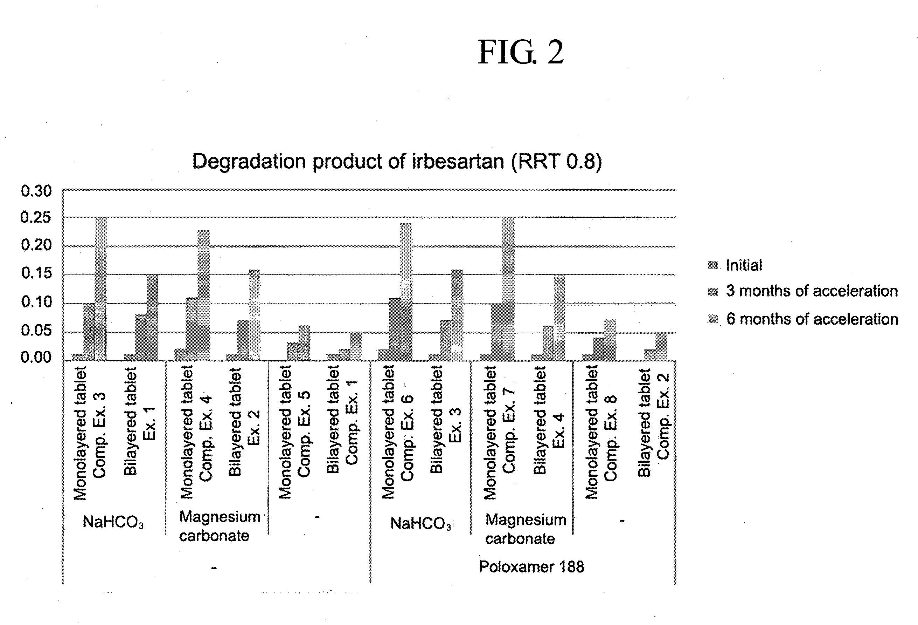

Furthermore, it was confirmed from Table 5 and Fig. 2 that configuration of bilayered tablets could improve the stability of formulations by inhibiting the interaction between a basic additive such as carbonates and irbesartan. More particularly, where a basic additive such as NaHCO₃ or magnesium carbonate is included in a formulation (Comparative Example 3-4) showed rapid increase in the amount of related compounds compared to where no basic additive is included in a formulation (Comparative Example 1-2), but Examples 1-4 in the form of bilayered complex formulations showed decreased amount of related compounds in spite of containing a basic additive to meet the requirement of the ICH guideline.

In summary, basic additives have problems in lowering the stability of irbesartan in spite of improvement on the stability of atovastatin. However, the inventive formulation can minimize the contact of inter-drugs or between a drug and a substance adversely affecting the stabilityof the drug, leading to improved stability in the preparation and storage of the complex formulation of irbesartan-atorvastatin.

### Experimental Example 2: Dissolution test

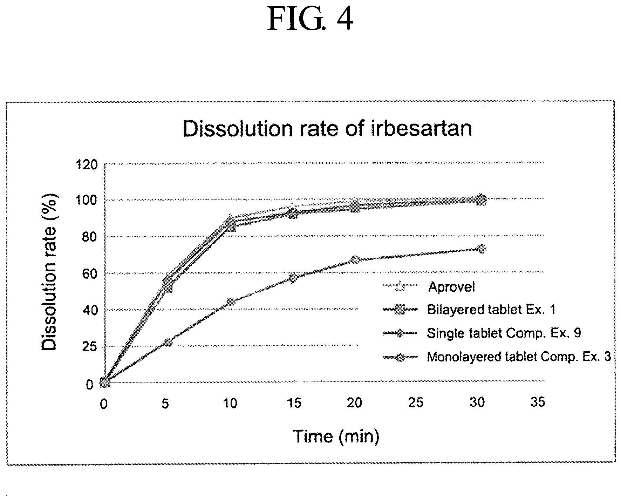

Comparative Example 3, Example 1, Comparative Example 9 and Aprovel 150 mg (a control drug, Sanofi-aventis) were tested using the dissolution test of 'irbesartan tablet' of the USP. The samples were taken at 5, 10, 15, 20 and 30 min after test initiation and measured for dissolution rates. The results are shown in Fig. 4.

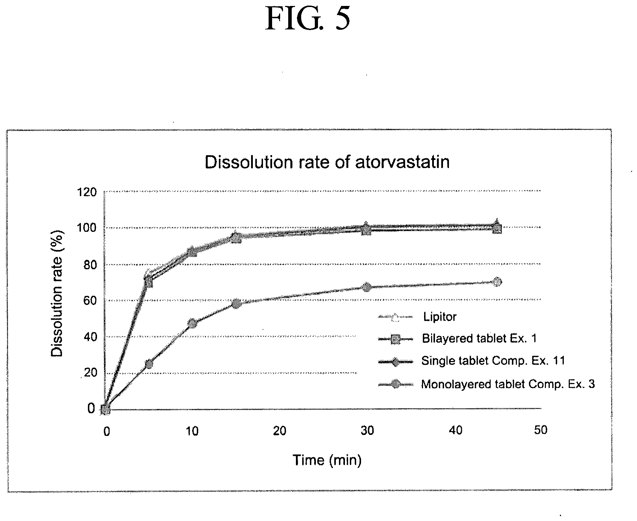

In addition, Comparative Example 3, Example 1, Comparative Example 9 and Lipitor 20 mg (a control drug, Pfizer) were tested using USP apparatus 2, in 900 mL of water with paddle speed of 50 rpm. The samples were taken at 5, 10, 15, 30 and 45 min and measured for dissolution rates. The results are shown in Fig. 5.

From the results of Figs. 4 and 5, it was found that monolayered formulation influenced the dissolution reduction of irbesartan and atorvastatin, while bilayered formulations did not influence the above compounds to show dissolution rates comparable to the control drug. Thus, the bilayered formulation in which two drugs are separated from each other would be preferable in preparation of complex formulations of irbesartan-atorvastatin for improving the dissolution rate.

### Example 3: Evaluation of saturation solubility of irbesartan

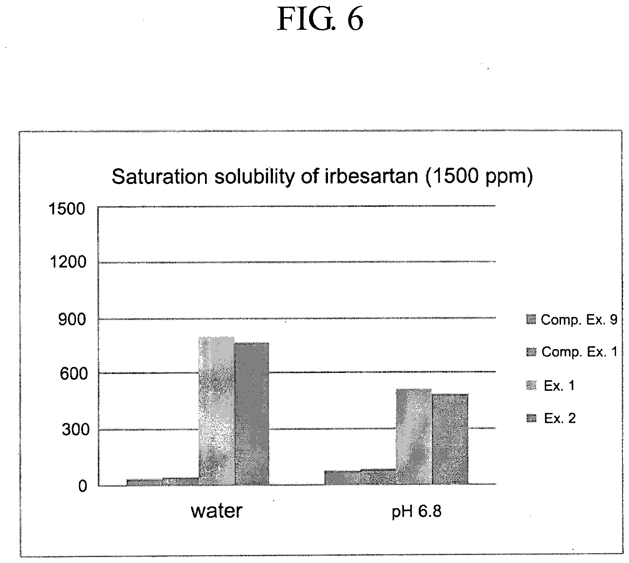

Comparative Example 1, Comparative Example 9 and Example 1 were measured for the saturation stability of irbesartan. The test was carried out using ten (10) tablets and USP apparatus 2, in 1000 mL of water and 1000 mL of pH 6.8 solution with paddle speed of 50 rpm. After 12 hours of test, sample solutions were taken and measured for their saturation solubility, and the results are shown in Fig. 6.

From the results of Fig. 6, it was revealed that the single tablet of irbesartan (Comparative Example 9) showed a low saturation solubility in water and pH 6.8 solution due to hydrophobicity of irbesartan, and also that the complex formulation of atorvastain containing no irbesartan and a basic additive (Comparative Example 1) showed a low saturation stability comparable to the single tablet of irbesartan, while the complex formulation containing a basic additive (Example 1) showed high increases in water and pH 6.8 solution. Thus, it was found that the basic additive improves the solubility of water-insoluble irbesartan.

### Experimental Example 4: Evaluation of bioavailability of irbesartan

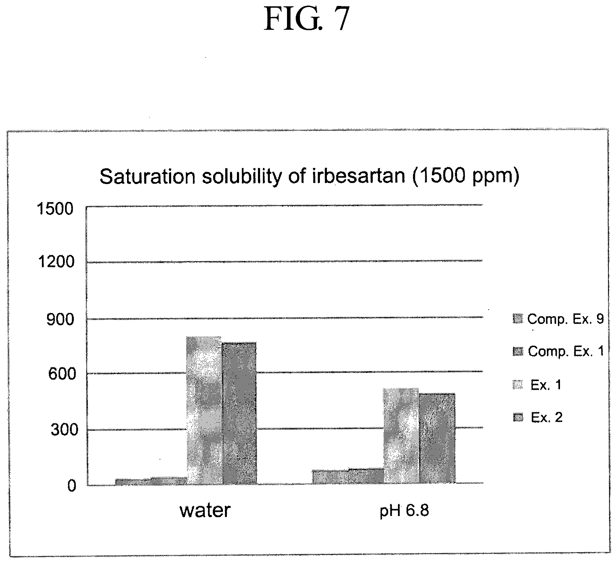

Example 1 and Comparative Example 9 were assessed for the bioavailability using beagle dogs. Six beagles were randomly cross-studied, and the results are shown in Table 7 and Fig. 7. Fig. 7 shows the calculated mean plasma concentration (mg/mL) versus time (hr) for irbesartan on a linear scale.

**<Table 7>**

| Pharmacokinetic parameters of irbesartan | | |
|---|---|---|
| | Irbesartan | |
| Parameters | Example 1 | Comparative Example 9 |
| AUC0-48 (ng·hr/mL) | 19677.4±5168.8 | 9760.7±6856.2 |
| Cₘₐₓ (ng/mL) | 13428.3±8016.0 | 5438.0±2656.6 |
| Tₘₐₓ (hr) | 1.1±0.5 | 0.7±0.3 |

As shown in Table 7 and Fig. 7, the complex formulation of irbesartan-atorvastatin containing a basic additive (Example 1) showed a higher bioavailability of irbesartan than the single formulation of irbesartan (Comparative Example 9), which was believed to be associated with the increase in solubility. Thus, it was found that a basic additive improves the solubility of irbesartan, and ultimately its bioavailability.

While the invention has been described with respect to the above specific embodiments, it should be recognized that various modifications and changes may be made to the invention by those skilled in the art which also fall within the scope of the invention as defined by the appended claims.

## Claims

1. A pharmaceutical formulation in the form of a tablet comprising:
a) a first layer containing irbesartan or a pharmaceutically acceptable salt thereof; and
b) a second layer containing an HMG-CoA reductase inhibitor and a basic additive, wherein the basic additive is contained in the second layer only; and wherein the basic additive is NaHCO₃, MgCO₃ or a mixture thereof.

2. The pharmaceutical formulation of claim 1, wherein the HMG-CoA reductase inhibitor is selected from the group consisting of rosuvastatin, lovastatin, atorvastatin, pravastatin, fluvastatin, pitavastatin, simvastatin, rivastatin, cerivastatin, velostatin, mevastatin, and pharmaceutically acceptable salts, precursors and mixtures thereof.

3. The pharmaceutical formulation of claim 2, wherein the HMG-CoA reductase inhibitor is atorvastatin.

4. The pharmaceutical formulation of claim 1, wherein the basic additive is included in an amount of 2 to 10 parts by weight based on 1 part of the HMG-CoA reductase inhibitor.

5. The pharmaceutical formulation of claim 1, wherein the basic additive is included in an amount of 0.2 to 10 parts by weight based on 1 part of irbesartan.

6. The pharmaceutical formulation of claim 1, wherein the second layer of the formulation further comprises a water-soluble diluent selected from the group consisting of mannitol, sucrose, lactose, sorbitol, xylitol, glucose, and mixtures thereof.

7. The pharmaceutical formulation of claim 1, wherein the second layer of the formulation further comprises disintegrants, binders, carriers, fillers, lubricants, rheology modifiers, crystallization retarders, solubilizers, coloring agents, pH modifiers, surfactants, emulsifiers, coating agents, or mixtures thereof.

8. The pharmaceutical formulation of claim 1, wherein the first layer of the formulation further comprises binders, disintegrants, lubricants, surfactants or mixtures thereof.

9. The pharmaceutical formulation of claim 1, wherein the formulation comprises irbesartan or a pharmaceutical acceptable salt thereof in an amount of 8 mg to 600 mg per unit dosage form.

10. The pharmaceutical formulation of claim 1, wherein the formulation comprises the HMG-CoA reductase inhibitor in an amount of 0.5 mg to 100 mg per unit dosage form.

11. A method for preparing the pharmaceutical formulation in the form of a tablet of any one of claims 1 to 11, comprising the steps of:
(i) granulating irbesartan or a pharmaceutically acceptable salt thereof to obtain granules for a first layer;
(ii) granulating a mixture of an HMG-CoA reductase inhibitor and a basic additive to obtain granules for a second layer; and
(iii) compressing the granules for the first layer and the second layer into a bilayered tablet,
wherein the basic additive is contained in the second layer only; and
wherein the basic additive is NaHCO₃, MgCO₃ or a mixture thereof.

## Patentansprüche

1. Pharmazeutische Rezeptur in Form einer Tablette, umfassend:
a) eine erste Schicht, enthaltend Irbesartan oder ein pharmazeutisch akzeptables Salz davon; und
b) eine zweite Schicht, enthaltend einen HMG-CoA-Reduktaseinhibitor und ein basisches Additiv, wobei das basische Additiv lediglich in der zweiten Schicht enthalten ist; und wobei das basische Additiv NaHCO₃, MgCO₃ oder ein Gemisch davon ist.

2. Pharmazeutische Rezeptur nach Anspruch 1, wobei der HMG-CoA-Reduktaseinhibitor ausgewählt ist aus der Gruppe, bestehend aus Rosuvastatin, Lovastatin, Atorvastatin, Pravastatin, Fluvastatin, Pitavastatin, Simvastatin, Rivastatin, Cerivastatin, Velostatin, Mevastatin und pharmazeutisch akzeptablen Salzen, Vorläufern und Gemischen davon.

3. Pharmazeutische Rezeptur nach Anspruch 2, wobei der HMG-CoA-Reduktaseinhibitor Atorvastatin ist.

4. Pharmazeutische Rezeptur nach Anspruch 1, wobei das basische Additiv in einer Menge von 2 bis 10 Gewichtsteilen, bezogen auf 1 Gewichtsteil des HMG-CoA-Reduktaseinhibitors, enthalten ist.

5. Pharmazeutische Rezeptur nach Anspruch 1, wobei das basische Additiv in einer Menge von 0,2 bis 10 Gewichtsteilen, bezogen auf 1 Teil Irbesartan, enthalten ist.

6. Pharmazeutische Rezeptur nach Anspruch 1, wobei die zweite Schicht der Rezeptur ferner ein wasserlösliches Verdünnungsmittel enthält, das ausgewählt ist aus der Gruppe, bestehend aus Mannitol, Saccharose, Laktose, Sorbitol, Xylitol, Glukose und Gemischen davon.

7. Pharmazeutische Rezeptur nach Anspruch 1, wobei die zweite Schicht der Rezeptur ferner Sprengmittel, Bindemittel, Träger, Füllstoffe, Schmiermittel, Mittel zum Modifizieren der Rheologie, Mittel zum Verlangsamen der Kristallisation, Löslichkeitsmittel, Kolorierungsmittel, pH-Modifikatoren, oberflächenaktive Substanzen, Emulgatoren, Beschichtungsmittel oder Mischungen davon enthält.

8. Pharmazeutische Rezeptur nach Anspruch 1, wobei die erste Schicht der Rezeptur ferner Bindemittel, Sprengmittel, Schmiermittel, oberflächenaktive Substanzen oder Gemische davon umfasst.

9. Pharmazeutische Rezeptur nach Anspruch 1, wobei die Rezeptur Irbesartan oder ein pharmazeutisch akzeptables Salz davon in einer Menge von 8 mg bis 600 mg pro Einheitsdosierform umfasst.

10. Pharmazeutische Rezeptur nach Anspruch 1, wobei die Rezeptur den HMG-CoA-Reduktaseinhibitor in einer Menge von 0,5 mg bis 100 mg pro Einheitsdosierungsform umfasst.

11. Verfahren zur Herstellung der pharmazeutischen Rezeptur in der Form einer Tablette nach einem der Ansprüche 1 bis 11, umfassend die Schritte:
(i) Granulieren von Irbesartan oder eines pharmazeutisch akzeptablen Salzes davon, um Granulate für eine erste Schicht zu erhalten;
(ii) Granulieren eines Gemisches eines HMG-CoA-Reduktaseinhibitors und eines basischen Additivs, um Granulate für eine zweite Schicht zu erhalten; und
(iii) Verpressen der Granulate für die erste Schicht und die zweite Schicht in eine zweischichtige Tablette,
wobei das basische Additiv lediglich in der zweiten Schicht enthalten ist; und
wobei das basische Additiv NaHCO₃, MgCO₃ oder ein Gemisch derselben ist.

## Revendications

1. Formulation pharmaceutique sous la forme d'un comprimé comprenant :
a) une première couche contenant de l'irbésartan ou un sel pharmaceutiquement acceptable de celui-ci ; et
b) une deuxième couche contenant un inhibiteur de l'HMG-CoA réductase et un additif basique, où l'additif basique est contenu dans la deuxième couche seulement ;
et où l'additif basique est le NaHCO₃, le MgCO₃ ou un mélange de ceux-ci.

2. Formulation pharmaceutique de la revendication 1, où l'inhibiteur de l'HMG-CoA réductase est sélectionné dans le groupe consistant en la rosuvastatine, la lovastatine, l'atorvastatine, la pravastatine, la fluvastatine, la pitavastatine, la simvastatine, la rivastatine, la cérivastatine, la vélostatine, la mévastatine et les sels, précurseurs et mélanges pharmaceutiquement acceptables de celles-ci.

3. Formulation pharmaceutique de la revendication 2, où l'inhibiteur de l'HMG-CoA réductase est l'atorvastatine.

4. Formulation pharmaceutique de la revendication 1, où l'additif basique est inclus à une quantité de 2 à 10 parties en poids pour 1 partie de l'inhibiteur de l'HMG-CoA réductase.

5. Formulation pharmaceutique de la revendication 1, où l'additif basique est inclus à une quantité de 0,2 à 10 parties en poids pour 1 partie d'irbésartan.

6. Formulation pharmaceutique de la revendication 1, où la deuxième couche de la formulation comprend en outre un diluant soluble dans l'eau sélectionné dans le groupe consistant en le mannitol, le sucrose, le lactose, le sorbitol, le xylitol, le glucose et des mélanges de ceux-ci.

7. Formulation pharmaceutique de la revendication 1, où la deuxième couche de la formulation comprend en outre des désintégrants, liants, véhicules, matières de charge, lubrifiants, modificateurs de la rhéologie, retardateurs de cristallisation, solubilisants, agents colorants, modificateurs du pH, agents tensio-actifs, émulsifiants, agents d'enrobage ou mélanges de ceux-ci.

8. Formulation pharmaceutique de la revendication 1, où la première couche de la formulation comprend en outre des liants, désintégrants, lubrifiants, agents tensio-actifs ou mélanges de ceux-ci.

9. Formulation pharmaceutique de la revendication 1, où la formulation comprend de l'irbésartan ou un sel pharmaceutiquement acceptable de celui-ci à une quantité qui va de 8 mg à 600 mg par forme galénique unitaire.

10. Formulation pharmaceutique de la revendication 1, où la formulation comprend l'inhibiteur de l'HMG-CoA réductase à une quantité qui va de 0,5 mg à 100 mg par forme galénique unitaire.

11. Procédé de préparation de la formulation pharmaceutique sous forme d'un comprimé de l'une quelconque des revendications 1 à 11, comprenant les étapes de :
(i) granulation de l'irbésartan ou d'un sel pharmaceutiquement acceptable de celui-ci pour obtenir des granules pour une première couche ;
(ii) granulation d'un mélange d'un inhibiteur de l'HMG-CoA réductase et d'un additif basique pour obtenir des granules pour une deuxième couche ; et
(iii) compression des granules de la première couche et la deuxième couche pour produire un comprimé à deux couches,
où l'additif basique est contenu dans la deuxième couche seulement ; et
où l'additif basique est le NaHCO₃, le MgCO₃ ou un mélange de ceux-ci.
